# DEMANDE DE BREVET EUROPEEN

(11) **EP 1 300 113 A1**
(43) Date de publication de la demande: **09.04.2003**
(21) Numéro de dépôt: 02292428.6
(22) Date de dépôt: 03.10.2002
(51) Int. Cl.: A61B 5/103

(54) **Dispositif de mesure de caractéristiques de la peau et procédé de mesure utilisant un tel dispositif**

(30) Priorité: 05.10.2001 FR 0112854
(71) Demandeur: Gharbi, Tijani, 25000 Besancon (FR); Wacogne, Bruno, 70190 Traitie Fontaine (FR); Pieralli, Christian, 25000 Besançon (FR); Humbert, Philippe Gérard Lucien, 25290 Ornans (FR); Marsaut, David, 25000 Besancon (FR)
(72) Inventeur: Gharbi, Tijani, 25000 Besancon (FR); Wacogne, Bruno, 70190 Traitie Fontaine (FR); Pieralli, Christian, 25000 Besançon (FR); Humbert, Philippe Gérard Lucien, 25290 Ornans (FR); Marsaut, David, 25000 Besancon (FR)
(74) Mandataire: Garel, Régis

(57) **Abrégé**

Dispositif de mesure d'au moins une caractéristique de la peau d'un sujet comprenant :
- un bras flexible (1) qui s'étend entre des première et deuxième extrémités (11,12) dont l'une au moins est reliée à un support (2), le bras flexible (1) comportant une face supérieure (13) et une face inférieure (14) pourvue d'un organe d'appui (3) destiné à être au contact de la peau du sujet,
- un dispositif de sollicitation (4) adaptés pour déformer en flexion le bras flexible (1) pour que ledit organe d'appui (3;9) applique une force prédéterminée sur la peau du sujet, et
- un dispositif de mesure du déplacement du bras flexible (1).

## Description

La présente invention se rapporte à un dispositif de mesure d'au moins une caractéristique de la peau d'un sujet et à un procédé de mesure utilisant un tel dispositif.

Il existe divers dispositifs de mesure qui fournissent des informations sur divers paramètres de la peau humaine. On connaît, notamment d'après le document FR-A-2 603 183, des instruments de mesure de la douceur de la peau et de l'indentation qui comportent un mécanisme de mesure complexe et fragile et qui fournit des valeurs mesurées relativement peu précises. Le manque de précision des valeurs mesurées est principalement dû au fait que ces instruments comprennent un élément rigide qui vient directement au contact de la peau du sujet en exerçant sur cette dernière une pression relativement élevée. Dès lors, ces instruments ne permettent pas de mesurer notamment les propriétés mécaniques de la couche supérieure de la peau, appelée le stratum corneum, avec une précision suffisante pour une exploitation fiable par exemple en dermatologie.

La présente invention a pour but de pallier les problèmes techniques mentionnées ci-dessus, en proposant un dispositif de mesure fiable, simple et qui fournisse des informations précises sur certaines propriétés mécaniques de la peau humaine.

A cet effet, l'invention a pour objet un dispositif de mesure d'au moins une caractéristique de la peau d'un sujet, caractérisé en ce qu'il comprend :
- au moins un bras flexible qui s'étend entre des première et deuxième extrémités dont l'une au moins est reliée à un support, le bras flexible comportant une face supérieure et une face inférieure pourvue d'un organe d'appui destiné à être au contact de la peau du sujet,
- des moyens de sollicitation adaptés pour déformer en flexion le bras flexible pour que ledit organe d'appui applique une force prédéterminée sur la peau du sujet, et
- des moyens de mesure du déplacement du bras flexible.

Dans des formes de réalisation préférées de l'invention, on a recours, en outre, à l'une et/ou à l'autre des dispositions suivantes :
- le bras flexible et le support sont réalisés dans une même plaque de silicium ;
- la plaque a une épaisseur comprise entre 360 µm et 390 µm ;
- l'organe d'appui est constitué par une pointe, les moyens de sollicitation étant adaptés pour que ladite pointe applique une force variable sur la peau du sujet, et les moyens de mesure étant agencés pour mesurer le déplacement du bras flexible en fonction de la force appliquée par ladite pointe sur la peau du patient ;
- l'organe d'appui est constitué par un micro-bille ayant un diamètre compris entre 0,5 mm et 2 mm et les moyens de sollicitation sont adaptés pour que ladite micro-bille applique une force constante sur la peau du sujet et les moyens de mesure étant agencés pour mesurer le déplacement du bras flexible en fonction des reliefs rencontrés par la micro-bille sur la peau du patient ;
- les moyens de sollicitation comprennent un élément piézoélectrique alimenté en tension par une source de tension, ledit élément piézoélectrique étant disposé sur l'une des faces supérieure et inférieure du bras flexible ;
- les moyens de sollicitation comprennent un générateur de champ électromagnétique qui agit directement sur le bras flexible ;
- les moyens de mesure comprennent une jauge de contrainte disposée sur le bras flexible, ladite jauge de contrainte étant destinée à être reliée à un calculateur ; et
- les moyens de mesure comprennent un système optique destiné à mesurer les déplacements relatifs d'une portion du bras flexible sur laquelle est disposé l'organe d'appui.

Par ailleurs, l'invention a également pour objet un procédé de mesure d'au moins une caractéristique de la peau d'un sujet au moyen d'un dispositif tel que défini ci-dessus, dans lequel on applique le support sur la peau, on actionne les moyens de sollicitation pour appliquer ladite force prédéterminée sur la peau, et on mesure le déplacement de l'organe d'appui.

D'autres caractéristiques et avantages de l'invention apparaîtront au cours de la description qui va suivre de plusieurs de ses formes de réalisation, données à titre d'exemples non limitatifs, en regard des dessins joints.

Sur les dessins :
- la figure 1 est une vue de dessous du dispositif conforme à l'invention selon une forme de réalisation ;
- la figure 2 est une vue en coupe verticale du dispositif représenté sur la figure 1 ;
- la figure 3 est une vue similaire à celle de la figure 2 selon une variante de réalisation du dispositif,
- la figure 4 est une vue de dessous du dispositif selon une autre forme de réalisation, et
- la figure 5 est une vue de dessus du dispositif conforme à l'invention selon encore une autre forme de réalisation.

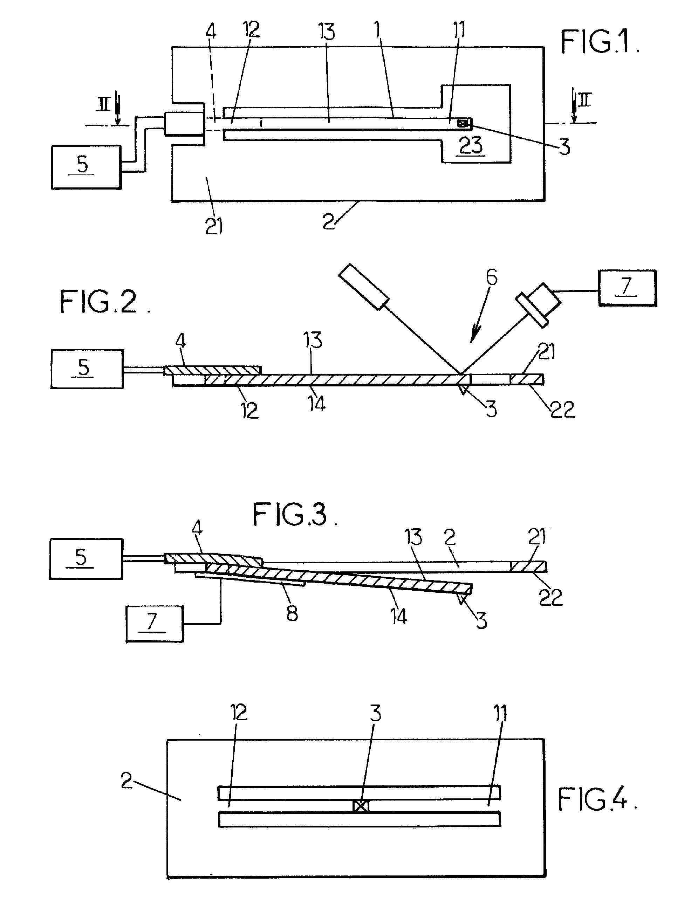

Le dispositif représenté sur la figure 1 est destiné à mesurer l'indentation in vivo de la peau, c'est-à-dire une compression de faible amplitude imposée à la peau. L'indentation de la peau permet de connaître avec précision ses propriétés mécaniques et permet donc indirectement l'analyse et la comparaison, par exemple, de l'effet de différents produits cosmétiques appliqués sur la peau d'un patient.

Certaines mesures doivent donc être faites sur la couche supérieure de la peau, appelée la couche cornée ou stratum corneum, ce qui implique de procéder à des mesures de très grande précision avec des dispositifs de mesure très sensibles.

A cet effet, le dispositif comprend un bras flexible 1 qui s'étend entre une première extrémité libre 11 et une deuxième extrémité 12 reliée à un support 2.

Le bras flexible 1 et le support 2 sont réalisés de préférence dans une plaque de silicium qui autorise des usinages très précis avec des dimensions très faibles, la plaque de silicium pouvant, par exemple, avoir une épaisseur comprise entre 360 µm et 390 µm.

Le bras flexible comprend une face supérieure 13 et une face inférieure 14 (figure 2) sur laquelle est rapportée, au niveau de la première extrémité libre 11, une pointe 3 destinée à venir au contact de la peau du patient.

Afin de réaliser des micro-indentations, de l'ordre de quelques micromètres, de la couche supérieure de la peau, le dispositif comprend également des moyens de sollicitation adaptés pour déformer sensiblement le bras flexible 1, de sorte que la pointe 3 applique une force prédéterminée, de l'ordre de quelques micronewtons, sur la peau du patient.

Comme représenté sur la figure 2, ces moyens de sollicitation comprennent un élément piezoélectrique 4 qui est fixé sur la face supérieure 13 du bras flexible 1 et éventuellement en partie sur le support 2. Cet élément piezoélectrique 4 est agencé pour permettre une déflection du bras flexible et donc un déplacement sensiblement vertical de la pointe 3 lorsque ledit élément piezoélectrique 4 est alimenté en tension par un générateur de tension 5.

Lorsque le générateur de tension 5 ne délivre aucune tension à l'élément piezoélectrique 4, les faces supérieure 13 et inférieure 14 du bras flexible 1 sont contenues respectivement dans un même plan que les faces supérieure 21 et inférieure 22 du support 2.

Le dispositif comporte également des moyens de mesure qui permettent de mesurer le déplacement relatif du bras flexible 1 ou plus précisément le déplacement relatif de l'extrémité libre 11 du bras lorsque la pointe 3 pénètre plus ou moins profondément dans la couche supérieure de la peau du patient. Ces moyens de mesure peuvent être constitués par un dispositif optique 6 qui envoie un faisceau de lumière en direction de l'extrémité libre 11 du bras flexible, le faisceau de lumière réfléchi étant ensuite analysé par un système optique approprié. Le système optique 6 peut être constitué par un interféromètre de type Fabry-Pérot comprenant une fibre optique dont l'extrémité de propagation de la lumière est pourvue d'un miroir formant le premier miroir de la cavité Fabry-Pérot tandis que le deuxième miroir de ladite cavité Fabry-Pérot est formé par une portion réfléchissante de l'extrémité libre 11 du bras flexible.

Ainsi, pour procéder à une mesure d'indentation de la peau d'un patient, il suffit de disposer la surface inférieure 22 du support 2 sur une zone choisie de la peau, de mesurer la position initiale de l'extrémité libre 11 du bras flexible qui comporte la pointe 3 par l'intermédiaire des moyens de mesure, d'appliquer ensuite une tension prédéterminée à l'élément piezoélectrique 4 afin que la pointe 4 exerce une force prédéterminée et connue sur la peau du patient puis de mesurer à nouveau le déplacement de l'extrémité libre 11 du bras.

Toutes les mesures recueillies par le système optique 6 sont envoyées à un calculateur 7 qui détermine en fonction de la configuration de la pointe sélectionnée, les propriétés mécaniques de la couche supérieure de la peau par la relation existant entre la force appliquée par la pointe 3 sur la peau et le déplacement relatif de l'extrémité libre 11 du bras qui porte ladite pointe. Les mesures peuvent être ensuite répétées pour des valeurs de tension différentes appliquées directement à l'élément piezoélectrique qui se déformera alors progressivement en modifiant ainsi la force appliquée par la pointe 3 sur la peau.

Bien entendu, on comprend que la relation entre la tension appliquée sur l'élément piezoélectrique et la force exercée par la pointe sur la peau, est fonction des propriétés intrinsèques et des dimensions du bras flexible. Ainsi, pour optimiser les mesures, il suffit de modifier la longueur et/ou l'épaisseur dudit bras flexible, et de procéder à des mesures préalables de la force exercée par la pointe 3 en fonction de la tension délivrée à l'élément piezoélectrique 4, pour pouvoir établir un tableau ou une courbe de correspondance entre ladite force exercée par la pointe et la tension à délivrer à l'élément piezoélectrique, ce tableau ou cette courbe de correspondance étant mémorisés par le calculateur 7.

Selon une autre variante de réalisation du dispositif, représentée sur la figure 3, les moyens de mesure du déplacement relatif du bras flexible comprennent une jauge de contrainte 8 directement disposée dans une zone de déformation du bras flexible 1. Dans l'exemple considéré sur la figure 3, la jauge de contrainte 8 est en partie disposée sur la jonction définie par le support 2 et l'extrémité 12 dudit bras flexible.

Ainsi, les forces exercées par la pointe 3 sur la peau du patient se traduisent par une pénétration de ladite pointe 3 dans la peau du patient, ce qui correspond à une certaine flexion du bras flexible qui est mesurée par la jauge de contrainte 8.

La jauge de contrainte 8 peut notamment être constituée d'une couche conductrice directement rapportée sur la face inférieure 14 du bras flexible 1. Les déformations d'allongement ou de contraction subies par la couche conductrice seront alors directement mesurées par une variation de la résistance d'un élément résistif en relief formé dans la couche conductrice et dont les extrémités sont reliées au calculateur 7.

Selon un autre mode de réalisation représenté sur la figure 4, les deux extrémités 11, 12 du bras flexible 1 sont reliées au support 2 en ne formant qu'une seule pièce avec ce dernier. Dans ce cas, la pointe 3 est disposée de préférence dans la zone de déformation maximale du bras flexible, c'est-à-dire à égale distance entre ses deux extrémités 11 et 12. Les moyens de sollicitation du bras flexible peuvent également se présenter sous la forme d'un élément piezoélectrique disposé au milieu de la face supérieure du bras flexible. Toutefois, on peut également prévoir que la déformation en flexion du bras flexible peut être opérée par le biais d'un générateur de champ électromagnétique qui délivre une certaine valeur de champ électromagnétique susceptible de déformer le bras flexible pour que ladite pointe 3 applique une force prédéterminée et connue sur la peau du patient. Selon ce mode de réalisation, les moyens de mesure peuvent être également constitués par un système optique approprié ou par une jauge de contrainte.

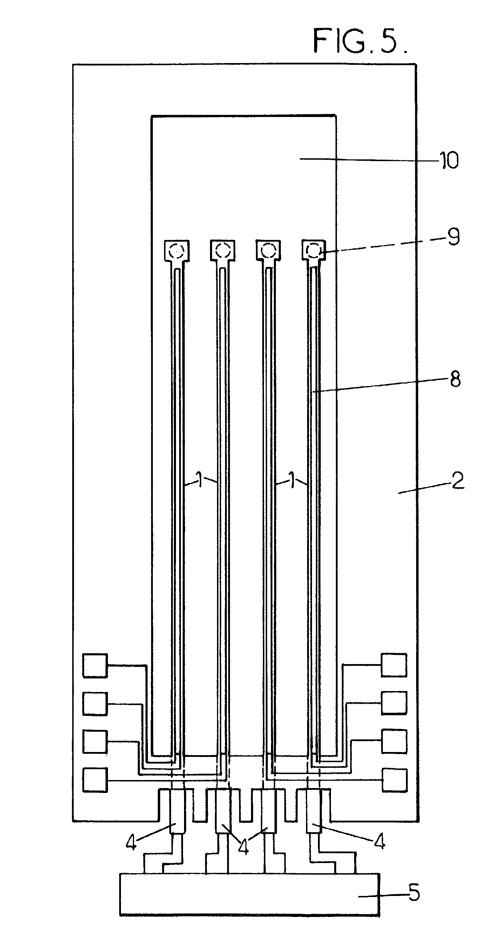

Le dispositif de mesure représenté sur la figure 5 est particulièrement adapté pour mesurer la rugosité locale de la peau et donc la douceur de la peau.

Ce dispositif comporte une pluralité de bras flexibles 1, parallèles entre eux, au nombre de quatre dans l'exemple considéré, qui sont tous reliés à un même support 2. Les bras flexibles 1 et le support 2 sont réalisés par exemple dans une plaque de silicium. Chaque bras flexible 1 comporte un élément piezoélectrique 4 alimenté en tension par un générateur de tension, et une jauge de contrainte 8 reliée à des contacts électriques eux-mêmes reliés à un calculateur qui mesure la déformation du bras flexible correspondant. Dans ce mode de réalisation, les différents organes d'appui destinés à être au contact de la peau sont constitués par des micro-billes 9 qui sont fixées sur les extrémités libres des bras flexibles. Ces micro-billes 9 sont destinées à être déplacées sur la peau du patient. A cet effet, le dispositif de mesure peut être directement déplacé manuellement sur la peau du patient ou alors être rapporté sur un système adéquat qui sera susceptible de déplacer le dispositif de mesure à vitesse constante sur la peau du patient.

Ainsi, pour procéder à des mesures de douceur de la peau, il suffit d'alimenter chaque élément piezoélectrique 4 avec une tension constante déterminée afin que les micro-billes 9 exercent une force constante connue sur la peau du patient et de déplacer ensuite le support 2 pour mesurer la déflexion de chaque bras flexible en fonction des micro-rugosités rencontrées par les micro-billes 9. Ces micro-billes peuvent, par exemple, avoir un diamètre compris sensiblement entre 0,5 mm et 2 mm.

Le support 2 peut être déplacé de telle manière que les bras flexibles 1 subissent une translation dans une direction qui leur est parallèle et, dans ce cas, la déformation relative des bras flexibles permet de mesurer directement les micro-rugosités de la peau. Toutefois, on peut également envisager de déplacer le support 2 de telle manière que les bras flexibles 1 subissent une translation dans une direction qui leur est perpendiculaire. Dans ce cas, les déformations des différents bras flexibles dans un même plan contenant le support 2 et les bras flexibles 1 et qui seraient dues aux forces adhésives exercées par la peau sur les micro-billes, seront directement mesurées à l'aide des jauges de contraintes 8 ou d'un dispositif optique approprié.

Par ailleurs, on peut également prévoir une ouverture 10 sur le dispositif de mesure afin d'avoir une vision directe de la surface de la peau du patient lors des mesures, cette ouverture étant délimitée par les extrémités libres des bras flexibles et le support 2.

## Revendications

1. Dispositif de mesure d'au moins une caractéristique de la peau d'un sujet, **caractérisé en ce qu'**il comprend :
- au moins un bras flexible (1) qui s'étend entre des première et deuxième extrémités (11,12) dont l'une au moins est reliée à un support (2), le bras flexible (1) comportant une face supérieure (13) et une face inférieure (14) pourvue d'un organe d'appui (3;9) destiné à être au contact de la peau du sujet,
- des moyens de sollicitation (4) adaptés pour déformer en flexion le bras flexible (1) pour que ledit organe d'appui (3;9) applique une force prédéterminée sur la peau du sujet, et
- des moyens de mesure (6;8) du déplacement du bras flexible (1).

2. Dispositif selon la revendication 1, dans lequel le bras flexible (1) et le support (2) sont réalisés dans une même plaque de silicium.

3. Dispositif selon la revendication 2, dans lequel la plaque a une épaisseur comprise entre 360 µm et 390 µm.

4. Dispositif selon l'une quelconque des revendications précédentes, dans lequel l'organe d'appui est constitué par une pointe (3), les moyens de sollicitation (4) étant adaptés pour que ladite pointe (3) applique une force variable sur la peau du sujet, et les moyens de mesure (6;8) étant agencés pour mesurer le déplacement du bras flexible (1) en fonction de la force appliquée par ladite pointe (3) sur la peau du patient.

5. Dispositif selon l'une quelconque des revendications 1 à 3, dans lequel l'organe d'appui est constitué par un micro-bille (9) ayant un diamètre compris entre 0,5 mm et 2 mm et les moyens de sollicitation sont adaptés pour que ladite micro-bille (9) applique une force constante sur la peau du sujet et les moyens de mesure (6;8) étant agencés pour mesurer le déplacement du bras flexible (1) en fonction des reliefs rencontrés par la micro-bille (9) sur la peau du patient.

6. Dispositif selon l'une quelconque des revendications précédentes, dans lequel les moyens de sollicitation comprennent un élément piézoélectrique (4) alimenté en tension par une source de tension (5), ledit élément piézoélectrique (4) étant disposé sur l'une des faces supérieure (13) et inférieure (14) du bras flexible.

7. Dispositif selon l'une ou l'autre des revendications 1 à 5, dans lequel les moyens de sollicitation comprennent un générateur de champ électromagnétique qui agit directement sur le bras flexible (1).

8. Dispositif selon l'une quelconque des revendications précédentes, dans lequel les moyens de mesure comprennent une jauge de contrainte (8) disposée sur le bras flexible (1), ladite jauge de contrainte (8) étant destinée à être reliée à un calculateur (7).

9. Dispositif selon l'une quelconque des revendications 1 à 7, dans lequel les moyens de mesure comprennent un système optique (6) destiné à mesurer les déplacements relatifs d'une portion du bras flexible sur laquelle est disposé l'organe d'appui (3;9).

10. Procédé de mesure d'au moins une caractéristique de la peau au moyen d'un dispositif selon l'une quelconque des revendications précédentes, dans lequel on applique le support (2) sur la peau, on actionne les moyens de sollicitation (4) pour appliquer ladite force prédéterminée sur la peau, et on mesure le déplacement de l'organe d'appui (3;9).
